(19) Europäisches Patentamt / European Patent Office / Office européen des brevets

(11) **EP 3 756 545 B1**

(12) **EUROPEAN PATENT SPECIFICATION**

(45) Date of publication and mention
of the grant of the patent:
**21.07.2021 Bulletin 2021/29**

(51) Int Cl.:
***A61B 5/1455*** (2006.01)

(21) Application number: **20180702.1**

(22) Date of filing: **18.06.2020**

(54) **METHOD OF MEASURING BLOOD OXYGEN SATURATION**

VERFAHREN ZUR MESSUNG DER SAUERSTOFFSÄTTIGUNG DES BLUTES

PROCÉDÉ DE MESURE DE LA SATURATION EN OXYGÈNE DANS LE SANG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.06.2019 JP 2019118227**

(43) Date of publication of application:
**30.12.2020 Bulletin 2020/53**

(73) Proprietor: **Phoenix Electric Co., Ltd.
Himeji-shi, Hyogo 679-2122 (JP)**

(72) Inventor: **NOGAWA, Masamichi
Komatsu-shi, Ishikawa 9230921 (JP)**

(74) Representative: **Horn Kleimann Waitzhofer
Patentanwälte PartG mbB
Ganghoferstrasse 29a
80339 München (DE)**

(56) References cited:
**US-A1- 2008 262 325    US-A1- 2019 053 745**

- **NORBERT STUBÁN ET AL: "Non-invasive calibration method for pulse oximeters", PERIODICA POLYTECHNICA ELECTRICAL ENGINEERING, vol. 52, no. 1-2, 22 July 2008 (2008-07-22), pages 91-94, XP055743768, ISSN: 0031-532X, DOI: 10.3311/pp.ee.2008-1-2.11**
- **NUR ANIDA JUMADI ET AL: "Development of theoretical oxygen saturation calibration curve based on optical density ratio and optical simulation approach", AIP CONFERENCE PROCEEDINGS, vol. 1883, 14 September 2017 (2017-09-14), pages 20024-1-20024-10, XP055743889, NEW YORK, US ISSN: 0094-243X, DOI: 10.1063/1.5002042**
- **FINE I: "The optical origin of the PPG signal", PROGRESS IN BIOMEDICAL OPTICS AND IMAGING, SPIE - INTERNATIONAL SOCIETY FOR OPTICAL ENGINEERING, BELLINGHAM, WA, US, vol. 9031, 26 January 2014 (2014-01-26), pages 903103-903103, XP060036225, ISSN: 1605-7422, DOI: 10.1117/12.2051228 ISBN: 978-1-5106-0027-0**
- **REDDY K A ET AL: "A Novel Calibration-Free Method of Measurement of Oxygen Saturation in Arterial Blood", IEEE TRANSACTIONS ON INSTRUMENTATION AND MEASUREMENT, IEEE SERVICE CENTER, PISCATAWAY, NJ, US, vol. 58, no. 5, 7 April 2009 (2009-04-07), pages 1699-1705, XP011251072, ISSN: 0018-9456**

EP 3 756 545 B1

**Description**

BACKGROUND OF THE INVENTION

Field of the Invention

The present invention relates to a method of measuring blood oxygen saturation of a person in a noninvasive manner.

Background Art

There has been conventionally developed a method of measuring blood oxygen saturation without injuring a human body (in a noninvasive manner). For example, Japan Laid-open Patent Application Publication No. 2014-117503 discloses a method of measuring oxygen saturation whereby accurate calculation of oxygen saturation is enabled with only one light incident position and only one light detecting position. US 2008/262325 A1 and US 2019/053745 A1 relate to methods of determining blood oxygen saturation.

SUMMARY OF THE INVENTION

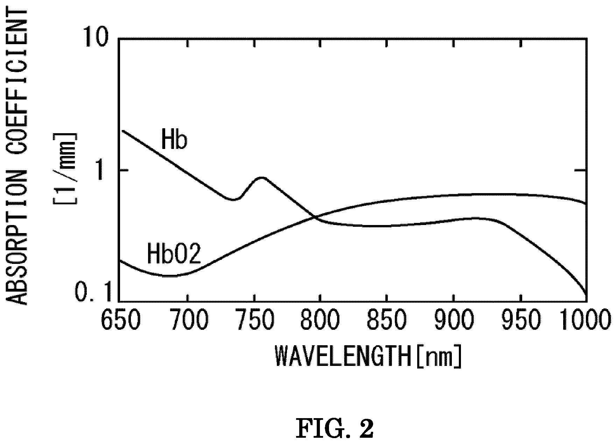

Blood oxygen saturation has been measured with light not only by the aforementioned method described in Japan Laid-open Patent Application Publication No. 2014-117503 but also by the following method. It should be noted that an instrument for measuring oxygen saturation in arterial blood with light is referred to as a pulse oximeter.
As shown in FIG. 2, by comparison between spectral absorption coefficient of oxyhemoglobin ($HbO_2$) formed when oxygen binds to hemoglobin and that of deoxyhemoglobin (Hb) formed when oxygen is released from oxyhemoglobin, it is understood that deoxyhemoglobin absorbs more light than oxyhemoglobin in the vicinity of red light (visible light) whereas oxyhemoglobin absorbs more light than deoxyhemoglobin in the vicinity of infrared light.
Oxygen saturation is calculated with the following formula based on oxyhemoglobin density and deoxyhemo-globin density in blood.

$$\text{Oxygen saturation = "oxyhemoglobin density" / "total hemoglobin density"} \times 100 \ (\%)$$

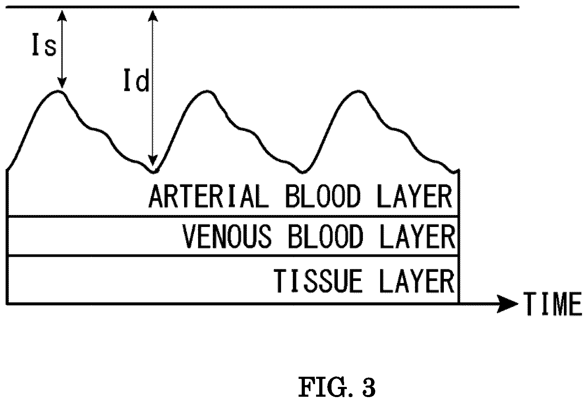

It should be noted that "total hemoglobin density" is a value approximately equal to the sum of oxyhemoglobin density and deoxyhemoglobin density.
It should be noted that oxygen saturation measured by the pulse oximeter is referred to as percutaneous oxygen saturation in arterial blood ($SpO_2$); oxygen saturation measured with collected arterial blood is referred to as arterial oxygen saturation ($SaO_2$); oxygen saturation in venous blood is referred to as venous oxygen saturation ($SvO_2$).
In measuring blood oxygen saturation with the pulse oximeter, light with a predetermined wavelength is irradiated on the human body and the intensity of light reflected by the human body is measured. Arterial blood pulsates with a large amplitude. Hence, as shown in FIG. 3, the intensity of light herein measured repeatedly increases and reduces in a periodic manner. Now, "$I_s$" is defined as the intensity of light obtained when optical path length is maximized in systole, whereas "$I_d$" is defined as the intensity of light obtained when the optical path length is minimized in diastole.
When a relation of absorbance is applied to Lambert-Beer law, the following relational expression is established between incident light $I_0$ (light incident into tissues) and detected light I (measured light) at a predetermined wavelength $\lambda$. It should be noted that, through the entire specification of the present application, the term "absorbance" refers to a dimensionless quantity indicating to what extent the intensity of light is weaken when the light transmits through an object.

$$OD^\lambda = \log (I_0^\lambda/I^\lambda) = \mu^\lambda L,$$

where OD: absorbance,
L: optical path length, and
$\mu$: absorption coefficient of tissues
Next, absorbance $\Delta OD$, in which only an arterial component is involved, is calculated by the following formula using $I_d$ and $I_s$ described above. Accordingly, elements of incident light $I_0$ can be canceled out.

$$\Delta OD = \log (I_d^\lambda / I_0^\lambda) - \log (I_s^\lambda / I_0^\lambda) = \log (I_d^\lambda / I_s^\lambda) = \mu_a^\lambda \Delta L,$$

where a: arterial blood,

d: diastole,

s: systole, and

$\Delta L$: variation in optical path length due to pulsation

Then, $\Delta OD$ is calculated for each of rays of light with two types of wavelengths ($\lambda_1$ and $\lambda_2$), and a ratio is calculated therebetween. Accordingly, variation ($\Delta L$) in optical path length due to pulsation can be canceled out.

$$\text{Ratio} = \Delta OD^{\lambda 1} / \Delta OD^{\lambda 2} = \log (I_d^{\lambda 1} / I_s^{\lambda 1}) / \log (I_d^{\lambda 2} / I_s^{\lambda 2})$$

$$= \mu_a^{\lambda 1} \Delta L / \mu_a^{\lambda 2} \Delta L = \mu_a^{\lambda 1} / \mu_a^{\lambda 2}$$

Finally, arterial oxygen saturation ($SaO_2$) can be calculated with the following calculation formula including calibration values A and B preliminarily measured and calculated with collected blood.

$$SaO_2 = A + B \times \text{Ratio}$$

However, the aforementioned conventional method of measuring blood oxygen saturation has had the following drawback. It is required for the conventional method to preliminarily measure and calculate calibration values A and B with preliminarily collected blood. Besides, the calibration values A and B slightly fluctuate among individuals. Because of this, in use of calibration values A and B preliminarily measured and calculated from blood of not a subject but anyone else, a numeric value of oxygen saturation herein calculated slightly deviates from a true value of oxygen saturation of the subject.

The present invention has been produced in view of the aforementioned drawback. It is an object of the present invention to provide a method of measuring blood oxygen saturation without necessity of using calibration values preliminarily measured and calculated.

According to an aspect of the present invention, a method of measuring blood oxygen saturation is provided. The method includes the following processing:

irradiating a radiation light containing a plurality of rays of light with wavelengths onto a predetermined part of a human body by a light source and measuring an intensity of each of the plurality of rays of light with wavelengths contained in the radiation light by a spectroscope when the radiation light is reflected by or transmits through the predetermined part of the human body;

calculating a measured ratio of a pulsatile component absorbance at each of the wavelengths except for a reference wavelength to a pulsatile component absorbance at the reference wavelength based on an intensity $I_d$ and an intensity $I_s$ of the each of the plurality of rays of light, the intensity $I_s$ defined as an intensity obtained when an optical path length is maximized in systole, the intensity $I_d$ defined as an intensity obtained when the optical path length is minimized in diastole;

calculating a theoretical ratio of the pulsatile component absorbance at the each of the wavelengths except for the reference wavelength to the pulsatile component absorbance at the reference wavelength based on giving an arbitrary initial value to a parameter required to calculate the blood oxygen saturation;

determining a numeric value of the parameter with an optimization algorithm such that the theoretical ratio and the measured ratio are matched; and thereafter

calculating the blood oxygen saturation based on the intensity $I_d$ and the intensity $I_s$.

Preferably, light diffusion equation is used in calculating the theoretical ratio.

Preferably, Nelder-Mead method is used in determining the numeric value of the parameter.

According to the present invention, it is possible to provide a method of measuring blood oxygen saturation without necessity of using a preliminarily set calibration value by measuring the blood oxygen saturation based on determining, with an optimization algorithm, a numeric value of a parameter required to calculate the blood oxygen saturation such that a measured ratio, calculated based on the intensity of each of a plurality of rays of light with wavelengths reflected by a human body, is matched with a theoretical ratio calculated based on giving an arbitrary initial value to the parameter.

BRIEF DESCRIPTION OF THE DRAWINGS

Referring now to the attached drawings which form a part of this original disclosure:

FIG. 1 is a schematic diagram showing a measuring device 10 according to an exemplary embodiment.

FIG. 2 is a chart showing a spectral absorption coefficient of oxyhemoglobin (HbO$_2$) and that of deoxyhemoglobin (Hb) formed when oxygen is released from oxyhemoglobin.

FIG. 3 is a chart showing chronological transition of a transmitted pulse wave signal.

FIG. 4 is an exemplary chart showing plots of measured Ratios, each of which is a Ratio of pulsatile component absorbance at each of wavelengths except for a reference wavelength $\lambda_t$ (e.g., $\lambda$ = 700 nm) to that at the reference wavelength $\lambda_t$.

FIG. 5 is an exemplary chart showing a condition that calculation results of theoretical ratios are plotted on the chart shown in FIG. 4.

FIG. 6 is an exemplary chart showing a condition that the theoretical ratios are matched with the measured ratios using optimization algorithm.

DETAILED DESCRIPTION OF EMBODIMENTS

(Structure of Measuring Device 10)

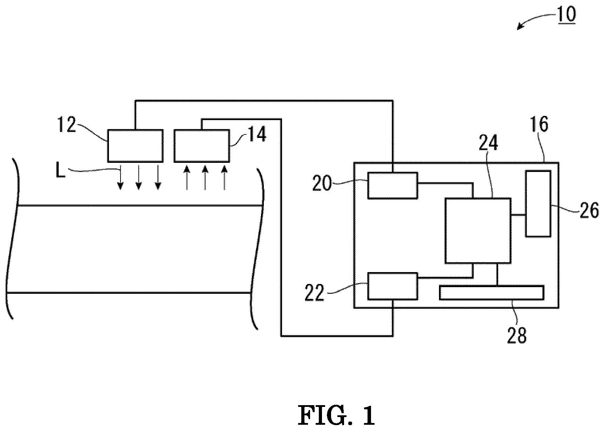

As shown in FIG. 1, a measuring device 10, which is a device for measuring blood oxygen saturation according to the present exemplary embodiment, is roughly composed of a light source 12, a spectroscope 14 and a control device 16.

The light source 12 is configured to irradiate radiation light L, containing a plurality of rays of light each having a predetermined wavelength, onto a predetermined part of a human body (e.g., fingertip, forehead, neck, chest, etc.). For example, a halogen bulb is used as the light source 12. Obviously, the light source 12 is not limited to the halogen bulb. Alternatively, a light emitting diode, an organic EL or so forth may be used as the light source 12. Besides, on an as-needed basis, a plurality of light sources 12 may be used while being configured to irradiate rays of light with different wavelengths.

The spectroscope 14 is configured to receive the radiation light L, which is emitted from the light source 12 and is then reflected by the predetermined part of the human body, and measure the intensity of each of rays of light with wavelengths contained in the radiation light L. In general, spectroscopes are configured to be capable of measuring the intensity of rays of light when the wavelengths of the rays of light fall in a predetermined range. Hence, it is required to select an appropriate one as the spectroscope 14 depending on a range of wavelengths of the rays of light contained in the radiation light L emitted from the light source 12. Besides, there are some types of spectroscopy to be used by the spectroscope 14, including a type with a prism, a type with an interferometer, and so forth. Any of the types of spectroscopy may be used as long as the selected one is suitable for a range of wavelengths of rays of light intended for intensity measurement. For example, as disclosed in Japan Laid-open Patent Application Publication No. 2003-275192, a type of spectroscopy with combination of "photodiode" and "bandpath filters" may be employed. Moreover, the spectroscope 14 may be configured to receive the radiation light L when the radiation light L transmits through the predetermined part of the human body.

Furthermore, a plurality of spectroscopes 14 may be disposed away from the predetermined part of the human body at different distances. This is because, in general, the spectroscope 14 disposed at short distance from the light source 12 is capable of obtaining information of tissue surfaces whereas the spectroscope 14 disposed at long distance from the light source 12 is capable of obtaining information of deep tissues. This is effective in that, by executing processing such as subtracting the data obtained by the short distance setting from the data obtained by the long distance setting, the information of deep tissues can be extracted without being affected by tissue surfaces. It should be noted that even when a plurality of light sources 12 are disposed away from the predetermined part of the human body at different distances, it is possible to achieve an advantageous effect similar to the above.

The control device 16 is configured to have functions of receiving a signal indicating the intensity of each of the rays of light with wavelengths measured by the spectroscope 14 and calculating blood oxygen saturation based on the intensity of each of the rays of light with wavelengths. The control device 16 according to the present exemplary embodiment includes, for instance, an electric power transmitter 20, a receiver 22, a controller 24, an electric power source 26 and a display 28.

The electric power transmitter 20 has a role of transmitting electric power for light emission to the light source 12. The magnitude of electric power transmitted thereto is controlled by the controller 24.

The receiver 22 has a role of receiving the intensity of each of the rays of light with wavelengths from the spectroscope 14 as an electric signal. The received electric signal is transmitted to the controller 24 as intensity data regarding each of the rays of light with wavelengths.

The controller 24 has a roll of controlling the entirety of the measuring device 10. As described above, the controller 24 determines the magnitude of electric power transmitted from the electric power transmitter 20 to the light source 12 and receives the intensity data regarding each of the rays of light with wavelengths from the receiver 22. Furthermore, the controller 24 calculates blood oxygen saturation and so forth in a measured part of the human body based on the received intensity data regarding each of the rays of light with wavelengths in a procedure to be described later.

The electric power source 26 has a role of transmitting electric power to the controller 24. A storage battery is employed as the electric power source 26 in order to enhance portability of the measuring device 10. Obviously, the electric power source 26 is equipped with an essential mechanism for receiving electric power from outside and storing the received electric power.

The display 28 has a role of displaying the blood oxygen saturation and so forth calculated by the controller 24 and a variety of information required to operate the measuring device 10. A heretofore known device, such as a liquid crystal screen, an organic EL screen or so forth, is used as the display 28.

Next, the procedure of calculating blood oxygen saturation and so forth by the controller 24 will be explained in a step-by-step manner.

First, absorbance $\Delta OD\lambda_1,...,\lambda_i,...,\lambda_N$ at each of wavelengths is calculated based on intensity $I_d$ and intensity $I_s$ of each of the rays of light contained in the radiation light L emitted from the light source 12. The intensity $I_s$ is defined as the intensity obtained when an optical path length is maximized in systole, whereas the intensity $I_d$ is defined as the intensity obtained when the optical path length is minimized in diastole.

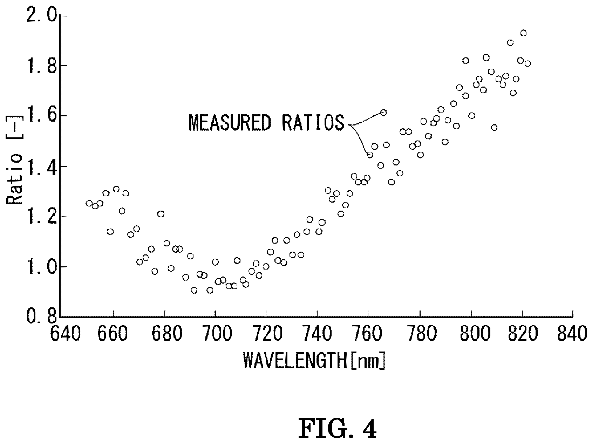

Next, any of the wavelengths is set as a reference wavelength $\lambda_t$ and calculation is done for a measured ratio of pulsatile component absorbance at each of the wavelengths except for the reference wavelength $\lambda_t$ (e.g., $\lambda = 700$ nm) to that at the reference wavelength $\lambda_t$. The term "pulsatile component absorbance" refers to absorbance in which not only arterial blood but also the amount of blood within tissues and venous oxygen saturation are taken into consideration (it should be noted that in the aforementioned pulse oximeter, only arterial blood is involved in pulsatile component absorbance). FIG. 4 shows an exemplary chart with plots of the measured ratios.

Through the calculations described so far, canceling out is done for difference in light intensity among wavelengths and difference in spectral sensitivity among the spectroscopes 14 each having unique spectral sensitivity. Besides, canceling out is done for difference in fluctuation of pulse wave amplitude among individuals, for whom blood oxygen saturation and so forth are measured.

Next, with light diffusion equation, calculation is done for a theoretical ratio of pulsatile component absorbance at each of the wavelengths except for the reference wavelength $\lambda_t$ to that at the reference wavelength $\lambda_t$, where the following numeric values are used as initial values for blood ratio (percentage) $\beta$ and venous oxygen saturation $SvO_2$, both of which are parameters (design variables):

ratio $\beta$ of blood in the entirety of blood and tissues = 5 (%);
ratio $\alpha$ of arteries in the entirety of arteries and veins = 30 (%);
arterial pulsatile component = 30 (%);
arterial oxygen saturation $SaO_2$ = 98 (%); and
venous oxygen saturation $SvO_2$ = 90 (%).

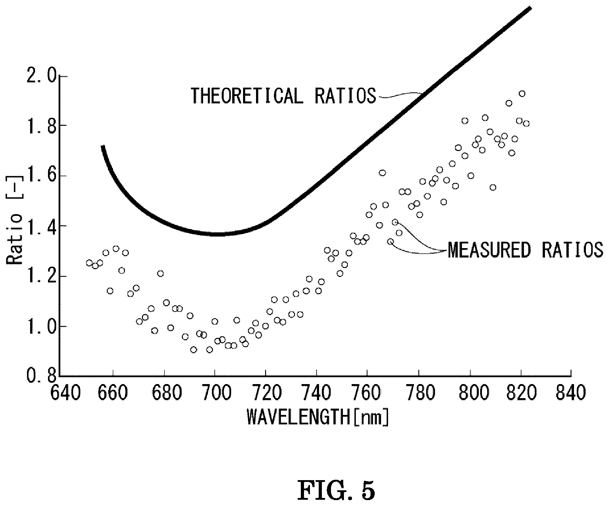

If results of calculating theoretical ratios are herein plotted on a chart, as shown in FIG. 5, there is a high possibility that the results deviate from the measured ratios calculated beforehand.

It should be noted that in calculating the theoretical ratios, Monte Carlo (MC) method, radiative transfer equation (RTE) or so forth may be used instead of the aforementioned light diffusion equation. However, it is preferred to use light diffusion equation because of its shortness in time for calculation. Besides, two types of solutions, a transmission-related solution and a reflection-related solution, are obtained by light diffusion equation. Hence, it is preferred to selectively use either of the solutions depending on the position of the spectroscope 14 described above. When it is intended to obtain more exact calculation results, MC method or RTE is selected.

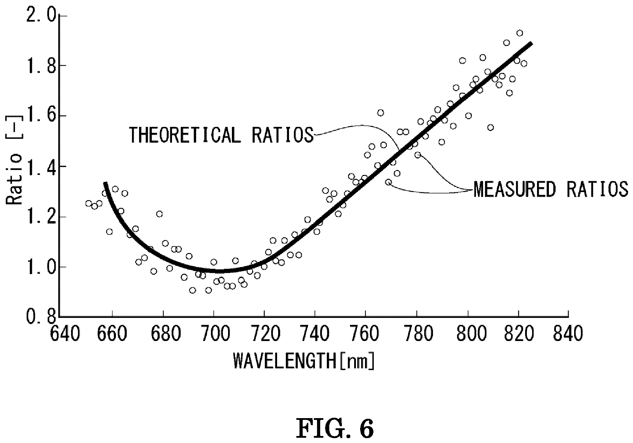

Thereafter, with an optimization algorithm such as Nelder-Mead method or differential evolution method, numeric values of the aforementioned parameters required to calculate blood oxygen saturation are determined such that the plots of the theoretical ratios are matched with those of the measured ratios (see FIG. 6).

Parameters herein required to calculate blood oxygen saturation are as follows.

(1) scattering coefficient: $(\mu s)^\lambda$

(2) absorption coefficient:

$(\mu_a^{Hb})^\lambda$ (absorption coefficient of deoxyhemoglobin);
$(\mu_a^{HbO2})^\lambda$ (absorption coefficient of oxyhemoglobin); and
$(\mu_a^t)^\lambda$ (absorption coefficient of tissues)

(3-1) entire absorption coefficient:

$$(\mu_a)^\lambda = \beta\,(\mu_a^t)^\lambda + (1 - \beta)\,(\mu_{ab})^\lambda$$

(3-2) absorption coefficient of entire blood (arterial blood and venous blood):

$$(\mu_{ab})^\lambda = \alpha\,(\mu_{aa})^\lambda + (1 - \alpha)\,(\mu_{av})^\lambda$$

(3-3) absorption coefficient of arterial blood:

$$(\mu_{aa})^\lambda = SaO_2\,(\mu_a^{HbO2})^\lambda + (1 - SaO_2)\,(\mu_a^{Hb})^\lambda$$

(3-4) absorption coefficient of venous blood:

$$(\mu_{av})^\lambda = SvO_2\,(\mu_a^{HbO2})^\lambda + (1 - SvO_2)\,(\mu_a^{Hb})^\lambda$$

(4) refractive index:
n = 1.4

(5) anisotropic scattering parameter:
g = 0.9

(6) number of photons in MC (Monte Carlo) method as an option in use of MC method:
50,000,000

(7) discretized domains in RTE (radiative transfer equation) as an option in use of RTE:

angular domain: 6
spatial domain: 5

Besides, in calculation with data of about 100 rays of light with wavelengths falling into a range of 650 nm to 820 nm, for instance, objective function f is set as follows. Obviously, the number of rays of light with wavelengths is not limited to the above. Alternatively, only several rays of light with wavelengths may be appropriately selected and calculation may be done with the selected rays of light with wavelengths.

[Math. 1]

$$f = \sum_{W=650}^{820} (Ratio_m - Ratio_t)^2$$

Moreover, constraints are set as follows:

$0 \le SvO_2 \le SaO_2$; and

$0 \le \beta$.

For example, with Nelder-Mead method, respective parameters (specifically, $\alpha$, $\beta$, $SvO_2$ and $SaO_2$) are calculated such that difference ($\Delta$Ratio) with respect to the ratio ($Ratio_t$) at the reference wavelength $\lambda_t$ is minimized.

Accordingly, blood ratio $\beta$ is derived for each of the individuals for whom blood oxygen saturation has been measured with the measuring device 10.

Besides, both of arterial oxygen saturation $SaO_2$ and venous oxygen saturation $SvO_2$ can be calculated.

Furthermore, tissue oxygen saturation $StO_2$ can be calculated as well.

Incidentally, occurrence of blood loss (bleeding) can be determined by continuously measuring ratio $\beta$ of blood in the entirety of blood and tissues.

Moreover, as an application of the ability to calculate both arterial oxygen saturation $SaO_2$ and venous oxygen saturation $SvO_2$, "tissue oxygen consumption" and "tissue oxygen extraction ratio" can be also calculated. Tissue oxygen consumption ($VO_2$) can be calculated with the following formula.

$$VO_2 = q \cdot k \cdot [Hb] \cdot (SaO_2 - SvO_2),$$

wherein q: blood flow rate [ml/min/100g];

k: 1.34 [ml/g] (amount of binding oxygen per 1g of hemoglobin); and

[Hb]: hemoglobin density [g/ml]

Tissue oxygen extraction ratio ($VO_2/DO_2$) can be calculated with the following formula and can be used to, for instance, assess brain tissue activity and muscle tissue activity.

$$VO_2/DO_2 = (q \cdot k \cdot [Hb] \cdot (SaO_2 - SvO_2)) / (q \cdot k \cdot [Hb] \cdot SaO_2) = 1 - SaO_2/SvO_2$$

It should be understood that the embodiment herein disclosed is illustrative only and is not restrictive in all aspects. It is intended that the scope of the present invention is indicated by the appended Claims.

It is, in particular, intended to provide a method of measuring blood oxygen saturation without necessity of using a preliminarily set calibration value. A radiation light, containing a plurality of rays of light with wavelengths, is irradiated on a predetermined part of a human body, and the intensity of each of the plurality of rays of light with wavelengths contained in the radiation light is measured by a spectroscope when the radiation light is reflected by or transmits through the predetermined part of the human body. A measured ratio of a pulsatile component absorbance at each of the wavelengths except for a reference wavelength to that at the reference wavelength is calculated based on an intensity $I_d$ and an intensity $I_s$ of the each of the plurality of rays of light, where the intensity $I_s$ is defined as an intensity obtained when an optical path length is maximized in systole while the intensity $I_d$ is defined as an intensity obtained when the optical path length is minimized in diastole. A theoretical ratio of the pulsatile component absorbance at the each of the wavelengths except for the reference wavelength to that at the reference wavelength is calculated based on giving an initial value to a parameter. A numeric value of the parameter is determined with an optimization algorithm such that the theoretical ratio and the measured ratio are matched. The blood oxygen saturation is calculated based on the intensity $I_d$ and the intensity $I_s$.

Reference Signs List

10    Measuring device,
12    Light source
14    Spectroscope
16    Control device
20    Electric power transmitter
22    Receiver
24    Controller
26    Electric power source

28   Display

**Claims**

1.  A method of measuring blood oxygen saturation, the method comprising:

    irradiating a radiation light containing a plurality of rays of light with wavelengths onto a predetermined part of a human body by a light source and measuring an intensity of each of the plurality of rays of light with wavelengths contained in the radiation light by a spectroscope when the radiation light is reflected by or transmits through the predetermined part of the human body;
    calculating a measured ratio of a pulsatile component absorbance at each of the wavelengths except for a reference wavelength to a pulsatile component absorbance at the reference wavelength based on an intensity $I_d$ and an intensity $I_s$ of the each of the plurality of rays of light, the intensity $I_s$ defined as an intensity obtained when an optical path length is maximized in systole, the intensity $I_d$ defined as an intensity obtained when the optical path length is minimized in diastole;
    calculating a theoretical ratio of the pulsatile component absorbance at the each of the wavelengths except for the reference wavelength to the pulsatile component absorbance at the reference wavelength based on giving an arbitrary initial value to a parameter required to calculate the blood oxygen saturation;
    determining a numeric value of the parameter with an optimization algorithm such that the theoretical ratio and the measured ratio are matched; and thereafter
    calculating the blood oxygen saturation based on the intensity $I_d$ and the intensity $I_s$.

2.  The method according to claim 1, wherein light diffusion equation is used in calculating the theoretical ratio.

3.  The method according to claim 1 or 2, wherein Nelder-Mead method is used in determining the numeric value of the parameter.

**Patentansprüche**

1.  Verfahren zur Messung einer Blutsauerstoffsättigung, wobei das Verfahren umfasst:

    Bestrahlen eines Strahlungslichts, das mehrere Lichtstrahlen mit Wellenlängen enthält, auf einen vorbestimmten Teil eines menschlichen Körpers durch eine Lichtquelle und Messen einer Intensität jeder der mehreren Lichtstrahlen mit Wellenlängen, die in dem Strahlungslicht enthalten sind, durch ein Spektroskop, wenn das Strahlungslicht von dem vorbestimmten Teil des menschlichen Körpers reflektiert wird oder durch diesen übertragen wird;
    Berechnen eines gemessenen Verhältnisses einer Absorption einer pulsierenden Komponente bei jeder der Wellenlängen mit Ausnahme einer Referenzwellenlänge zu einer Absorption einer pulsierenden Komponente bei der Referenzwellenlänge auf der Grundlage einer Intensität $I_d$ und einer Intensität $I_s$ jedes der mehreren Lichtstrahlen, wobei die Intensität $I_s$ als eine Intensität definiert ist, die erhalten wird, wenn eine optische Weglänge in der Systole maximiert ist, und die Intensität $I_d$ als eine Intensität definiert ist, die erhalten wird, wenn die optische Weglänge in der Diastole minimiert ist;
    Berechnen eines theoretischen Verhältnisses der Absorption der pulsierenden Komponente bei jeder der Wellenlängen mit Ausnahme der Referenzwellenlänge zu der Absorption der pulsierenden Komponente bei der Referenzwellenlänge auf der Grundlage des Festlegens eines willkürlichen Anfangswertes als ein Parameter, der zum Berechnen der Blutsauerstoffsättigung erforderlich ist;
    Bestimmen eines numerischen Wertes des Parameters mit einem Optimierungsalgorithmus, so dass das theoretische Verhältnis und das gemessene Verhältnis übereinstimmen; und anschließend
    Berechnen der Blutsauerstoffsättigung basierend auf der Intensität $I_d$ und der Intensität $I_s$.

2.  Verfahren nach Anspruch 1, wobei zur Berechnung des theoretischen Verhältnisses die Lichtdiffusionsgleichung verwendet wird.

3.  Verfahren nach Anspruch 1 oder 2, wobei das Nelder-Mead-Verfahren bei der Bestimmung des numerischen Wertes des Parameters verwendet wird.

**Revendications**

1. Procédé de mesure de la saturation en oxygène du sang, le procédé comprenant :

   l'irradiation d'une lumière de rayonnement contenant plusieurs rayons de lumière avec des longueurs d'onde sur une partie prédéterminée d'un corps humain par une source de lumière et la mesure d'une intensité de chacun des plusieurs rayons de lumière avec des longueurs d'onde contenus dans la lumière de rayonnement par un spectroscope lorsque la lumière de rayonnement est réfléchie par ou transmise à travers la partie prédéterminée du corps humain ;
   le calcul d'un rapport mesuré d'une absorbance de composant pulsatile à chacune des longueurs d'onde à l'exception d'une longueur d'onde de référence sur une absorbance de composant pulsatile à la longueur d'onde de référence sur la base d'une intensité $I_d$ et d'une intensité $I_s$ de chacun des plusieurs rayons de lumière, l'intensité $I_s$ étant définie comme une intensité obtenue lorsqu'une longueur de chemin optique est maximisée en systole, l'intensité $I_d$ étant définie comme une intensité obtenue lorsque la longueur de chemin optique est minimisée en diastole ;
   le calcul d'un rapport théorique de l'absorbance de composant pulsatile à chacune des longueurs d'onde à l'exception de la longueur d'onde de référence sur l'absorbance de composant pulsatile à la longueur d'onde de référence sur la base de l'attribution d'une valeur initiale arbitraire à un paramètre requis pour calculer la saturation en oxygène du sang ;
   la détermination d'une valeur numérique du paramètre avec un algorithme d'optimisation de telle sorte que le rapport théorique et le rapport mesuré correspondent ; et ensuite
   le calcul de la saturation en oxygène du sang sur la base de l'intensité $I_d$ et de l'intensité $I_s$.

2. Procédé selon la revendication 1, dans lequel l'équation de diffusion de la lumière est utilisée pour calculer le rapport théorique.

3. Procédé selon la revendication 1 ou 2, dans lequel la méthode de Nelder-Mead est utilisée pour déterminer la valeur numérique du paramètre.

FIG. 1

FIG. 2

FIG. 3

FIG. 4

FIG. 5

FIG. 6

**EP 3 756 545 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2014117503 A
- US 2008262325 A1
- US 2019053745 A1
- JP 2003275192 A